# EUROPÄISCHE PATENTSCHRIFT

(11) **EP 0 401 158 B1**
(45) Veröffentlichungstag und Bekanntmachung des Hinweises auf die Patenterteilung: **28.02.1996**
(21) Anmeldenummer: 90810353.4
(22) Anmeldetag: 11.05.1990
(51) Int. Cl.: A61B 17/22

(54) **Katheteranordnung mit einem Führungsdraht sowie Verfahren zur Herstellung eines solchen Führungsdrahtes**
Catheter apparatus with a guide wire and method for the production of such a guide wire
Cathéter pourvu d'un fil de guidage ainsi que méthode de fabrication d'un tel fil de guidage

(30) Priorität: 01.06.1989 CH 2056/89
(43) Veröffentlichungstag der Anmeldung: 05.12.1990
(73) Patentinhaber: SCHNEIDER (EUROPE) AG, CH-8180 Bülach (CH)
(72) Erfinder: Niederhauser, Werner, CH-8046 Zürich (CH); Meier, Bernhard, CH-1234 Vessy (CH); Bannwart, Urs, CH-8303 Bassersdorf (CH)
(74) Vertreter: Groner, Manfred

(56) Entgegenhaltungen:
- EP-A- 267 539
- EP-A- 0 145 489
- EP-A- 0 259 945
- EP-A- 0 279 959
- EP-A- 0 316 796
- GB-A- 2 127 294
- US-A- 3 433 226
- US-A- 4 832 047
- US-A- 4 854 325
- Werbeschrift der Schneider Medintag AG: "New Kaltenbach Long Wire"

## Beschreibung

Die Erfindung betrifft eine Katheteranordnung nach dem Oberbegriff des unabhängigen Patentanspruchs 1. Die Erfindung betrifft ebenfalls ein Verfahren zur Herstellung eines Führungsdrahtes für eine Katheteranordnung nach Patentanspruch 1.

Führungsdrähte sind beispielsweise durch die EP-A-0 279 959, EP-A-0 145 489, US-A-4,832,047 und die GB-A-2 127 294 bekannt geworden. Bei diesen ist die distale Oeffnung der Spiralfeder von einer Spitze abgedeckt, die durch Löten oder Schweissen gebildet wurde, und die in der Regel die Feder mit einem in dieser verlaufendem Draht bildet. Durch die EP-A-1 316 796 ist ein Katheter bekannt geworden, der einen Draht mit einer kugelförmigen Spitze aufweist. Die Spitze wird zu einer Engstelle gebracht und mit Ultraschallenergie bewegt, so dass Material der Engstelle pulverisiert wird. Die Spitze muss in der Regel gekühlt und das abgearbeitete Material weggespült werden.

Durch eine Werbeschrift der Schneider Medintag AG ist unter der Bezeichnung "New Kaltenbach Long Wire" ein Führungsdraht bekannt geworden, dessen Spitze kugelförmig ausgebildet ist und einen Durchmesser aufweist, der grösser ist als der Durchmesser der Spiralfeder des Schaftes unmittelbar hinter der Spitze.

Der Erfindung liegt die Aufgabe zugrunde, eine Katheteranordnung der genannten Gattung zu schaffen, mit der die Rekanalisation insbesondere koronarer, teilweise oder vollständig veschlossener chronischer Okklusionen insbesondere der Korronarartherie bei vergleichsweise einfacher Handhabung mit besseren Erfolgsaussichten als bisher durchgeführt werden kann. Die Aufgabe ist durch die Erfindung gemäss Anspruch 1 gelöst. Vorteilhafte Weiterbildungen der Erfindung ergeben sich aus den Ansprüchen 2 bis 16. Vorteilhafte Verfahren zur Herstellung eines Führungsdrahtes ergeben sich aus den Ansprüchen 17 bis 19.

Versuche haben gezeigt, dass der Führungsdraht in Verbindung mit einem an sich bekannten Ballonkatheter einfach, sicher und kostengünstig ist. Die erfindungsgemässe Anordnung besitzt eine höhere Erfolgsrate vermutlich infolge ihrer besseren Durchdringfähigkeit und einer Verminderung des Risikos eines sogenannten "subinitinalen" Durchgangs. Die Anordnung eignet sich insbesondere für die Behandlung chronischer Okklusionen.

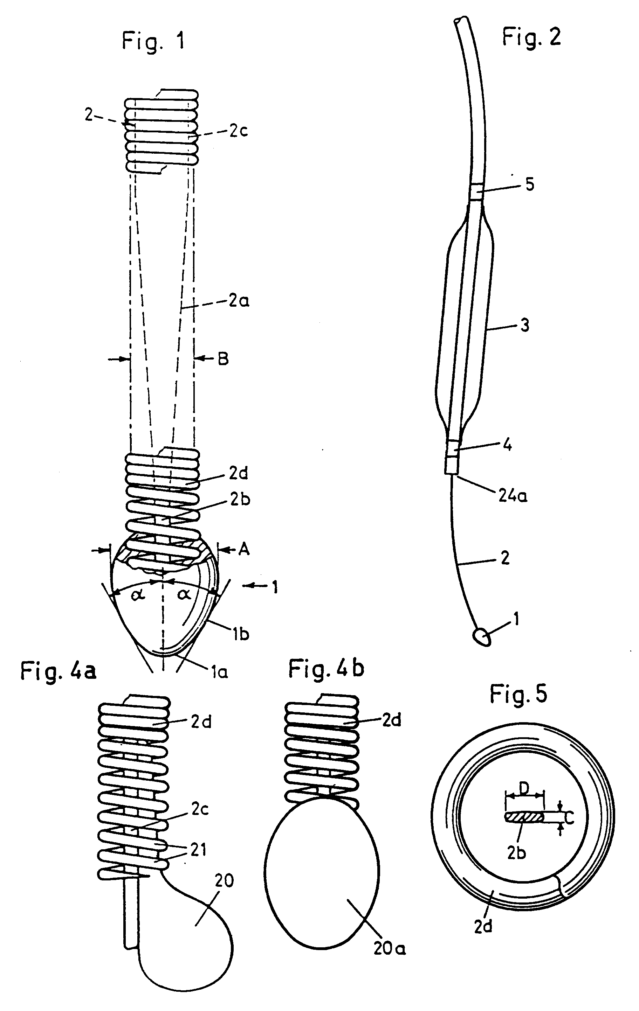

Der in das zu behandelnde Gefäss eingeführte flexible Führungsdraht ist eine besonders stabile Führung für den Ballonkatheter. Eine vorzugsweise etwa 1 mm breite olivenförmige Spitze am distalen Ende des Führungsdrahtes vermindert eine unbeabsichtigte Beschädigung der Gefässwand. Es hat sich gezeigt, dass ein sogenannter "high profile" Ballon auf einem im Anspruch 1 erwähnten Führungsdraht durch eine enge Stenose leichter vorgeschoben werden kann, als ein sogenannter "low profile" Ballon auf einem bekannten Führungsdraht. In allen untersuchten Fällen war es möglich, den Ballonkatheter bereits beim ersten Mal mit der endgültigen Grösse auf einem eingesetzten Führungsdraht einzuführen. Eine schrittweise Dilatation mit unterschiedlichen Kathetern ist somit nicht erforderlich. Denkbar ist auch eine Verwendung der erfindungsgemässen Katheteranordnung bei nicht totalen koronaren Verengungen. Ein Ausführungsbeispiel der Erfindung wird nachfolgend anhand der Figuren 1 bis 5 näher erläutert. Es zeigen:
- Fig. 1: in stark vergrössertem Massstab eine Ansicht eines teilweise geschnittenen erfindungsgemässen Führungsdrahtes,
- Fig. 2: schematisch eine Ansicht einer erfindungsgemässen Katheteranordnung,
- Fig. 3a bis 3f: schematisch die einzelnen Schritte bei er Behandlung einer Okklusion,
- Fig. 4a und 4b: schematische Darstellungen des distalen Endes des Führungsdrahtes zur Erläuterung des Verfahrens zur Herstellung der Sonde,
- Fig. 5: einen Querschnitt durch den Führungsdraht im Bereich in dem er flachgeschlagen ist,
- Fig. 6: eine schematische Darstellung einer weiteren Ausführung einer erfindungsgemässen Katheteranordnung, und
- Fig. 7: eine teilweise geschnittene Seitenansicht eines Antriebs der Anordnung nach Figur 6.

Der in Fig. 1 gezeigte Führungsdraht besitzt einen Schaft 2 mit einem teilweise teflonbeschichteten Draht 2c aus rostfreiem Stahl. Am distalen Ende ist der Draht 2c in einem Bereich 2a konisch verjüngt. Am vorderen Ende des Bereichs 2a ist der Stahldraht 2c in einem Bereich 2b auf einer Länge von etwa 2 cm flachgeschlagen. Durch das genannte Flachschlagen des Stahldrahtes 2 am distalen Ende wird die Torsionssicherheit bei einer Rotation des Katheters wesentlich erhöht. Ebenfalls wird die Ermüdungsgefahr kleiner. Die Gefahr eines Torsionsbruchs des Drahtes 2c an seinem distalen Ende wird somit durch das Flachschlagen wesentlich verringert. Versuche haben zudem gezeigt, dass damit die Zugfestigkeit des Drahtes 2c etwa verdoppelt werden kann. Zumindest im Bereich 2b ist der Schaft 2 nicht mit Teflon beschichtet.

Am vorderen Ende des Schaftes 2 ist eine olivenförmige Spitze 1 ausreissfest befestigt. Die Spitze 1 besteht vorzugsweise aus einer Silberlegierung, die röntgenographisch gut sichtbar ist. Der grösste Durchmesser A der Spitze 1 beträgt vorzugsweise 1 mm. Das vordere abgerundete Ende 1a der Spitze 1 führt zu einer Flanke 1b, in welcher die Spitze 1 vergleichsweise wenig gewölbt ist. Der Winkel dieser rotationsförmigen Flanke 1b zur Längsachse des Katheters beträgt beispielsweise etwa 30° (Winkel α).

Der Führungsdraht besitzt eine teilweise teflonbeschichtete Feder 2d, die vorzugsweise aus einem röntgensichtbaren Material z.B. Wolfram, Platin oder Gold besteht. Solche Federn sind an sich bekannt. Damit der Führungsdraht am distalen Ende gut flexibel ist, ist die Feder 2d hinter der Spitze 1 auf einer Länge von etwa 3 cm nicht beschichtet.

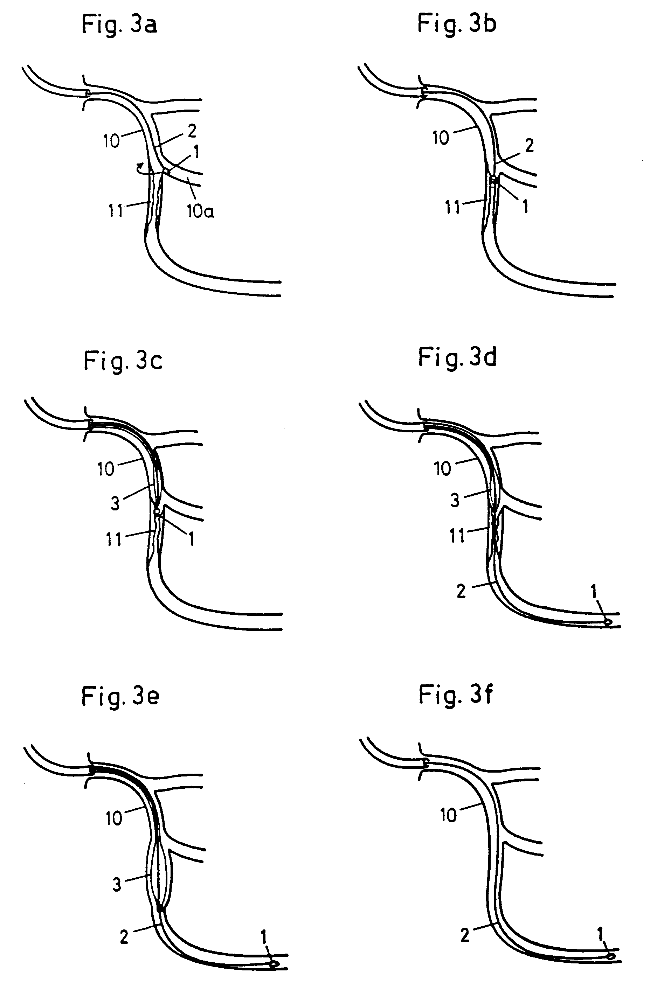

Die in Fig. 2 dargestellte Katheteranordnung umfasst einen Führungsdraht gemäss Fig. 1 sowie einen Ballonkatheter 3 mit einer distalen Oeffnung 24a, durch welche der Führungsdraht von vorne eingeschoben ist. Der Führungsdraht kann von Hand an seinem proximalen Ende axial im Gefäss verschoben sowie rotiert werden. Die Spitze 1 folgt diesen Bewegungen. Die Figuren 3a bis 3f zeigen jeweils ein Gefäss 1'0 mit einem Seitenast 10a und einer Okklusion 11. Gemäss den Figuren 3a und 3b wird die Spitze 1 eines am distalen Ende vorgebogenen Führungsdrahtes vor die Okklusion 11 geführt. Gemäss Fig. 3c wird nun der Ballon 3 des Ballonkatheters ebenfalls vorgeschoben, bis der Ballon unmittelbar vor der Spitze 1 vor der Okklusion 11 liegt. Durch den Ballonkatheter ist nun der Führungsdraht an seinem distalen Enden zusätzlich versteift, so dass die Spitze 1 nun gemäss Fig. 3d mit besonders grosser Schubkraft durch die Okklusion 11 hindurchgeschoben werden kann. Je näher der Ballon 3 bei der Spitze 1 ist, um so grösser ist die durch axiale Verschiebung des Führungsdrahtes ausübbare Schubkraft. Die Versteifung des distalen Endes des Führungsdrahtes ist somit auf einfache Weise veränderbar. Der geeignete plazierte Ballonkatheter unterstützt somit die Bewegung der Spitze 1. In die nun zumindest teilweise rekanalisierte Okklusion kann nun gemäss Fig. 3e der Ballon in diese Engstelle eingeführt werden. Durch eine kurze Füllung des Ballonkatheters mit Flüssigkeit unter Druck wird nun die Okklusion wieder durchgängig gemacht. Wie bereits erwähnt kann dieser Ballon bereits die dazu geeignete Grösse aufweisen. Dank der Spitze 1 können die Bewegungen des distalen Endes des Führungsdrahtes röntgenographisch besonders gut und deutlich durch den behandelnden Arzt verfolgt werden.

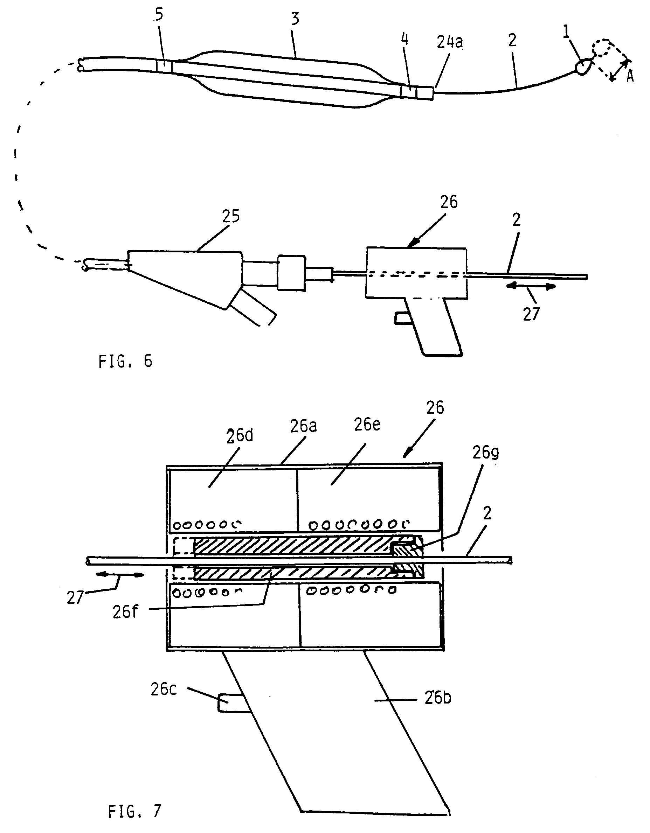

Anhand der Figuren 4a und 4b soll das erfindungsgemässe Verfahren zur Herstellung eines Führungsdrahtes erläutert werden. Der Draht 2c wird durch die Feder 2d geschoben, bis sein vorderes Ende, wie in Fig. 4a gezeigt, vorne aus der Feder 2d herausragt. Die vordersten Windungen 21 der Feder 2d sind hierbei axial gestreckt. Zwischen Feder 2d und Draht 2c wird nun ein Silberlotstück 20 eingesetzt. Nun wird mit einem Heizstrom das Silberlotstück 20 erwärmt bis es schmilzt. Sobald das Silberlotstück 20 zwischen die Windungen 21 geflossen ist und etwa die in Fig. 4b gezeigte Form angenommen hat, wird der Heizstrom weggenommen, worauf das Lot bzw. die Spitze 20a sofort erstarrt. Die Spitze 20a wird nun geschliffen, bis sie die geeignete olivenförmige Form besitzt. Das vordere Ende des Drahtes 2c sowie das vordere Ende der Feder 2d sind somit fest mit der Spitze 1 verbunden.

Vorzugsweise wird das vordere Ende des Drahtes 2c vor dem Aufbringen der Spitze 1 auf einer Länge von vorzugsweise etwa 2 cm flachgeschlagen, so dass der Draht 2c hier etwa den in Fig. 5 gezeigten Querschnitt besitzt. Die Dimension D beträgt hier vorzugsweise etwa 0,12 mm und die Dimension C vorzugsweise etwa 0,03 mm. Der Durchmesser des noch nicht flachgeschlagenen Drahtes 2c in diesem Bereich beträgt etwa 0,07 mm. Der Durchmesser des Drahtes 2c beträgt mit Ausnahme des konisch verjüngten Bereichs konstant etwa 0,5 mm. Dies ist auch etwa der Durchmesser der Feder 2d.

Das distale Ende des Führungsdrahtes kann gerade oder vorgebogen sein. In letztem Fall ist der Führungsdraht steuerbar, d.h. die Richtung der Spitze 1 kann durch eine Rotation des Führungsdrahtes um seine Längsrichtung geändert werden. In Fig. 3a wird durch eine solche Rotation verhindert, dass der Führungsdraht bzw. die Spitze 1 in den Seitenast 10a einfährt. Nachdem die Spitze 1 gebildet ist, wird das proximale Ende der Feder 2d mit dem Draht 2c verlötet. Die Länge der Feder 2d beträgt etwa 20-25 cm und entsprechend lang ist der sich konisch verjüngende Bereich 2a des Drahtes 2c. Die Feder 2d ist somit an einem Ende mit dem Draht 2c und am anderen Ende mit der Spitze 1 verbunden.

Die Katheteranordnung nach den Fig. 6 und 7 weist einen oben beschriebenen Ballonkatheter 3, einen bekannten Y-Verbinder 25 und einen Antrieb 26 auf, mit dem der Schaft 2 mit der Spitze 1 in den Richtungen des Doppelpfeils 27 vor und zurück bewegt werden kann, um dadurch ein Durchstossen einer Okklusion zu erleichtern.

Der Antrieb 26 weist ein Gehäuse 26a auf, an dem ein Handgriff 26b angebracht ist. Im Gehäuse 26a sind zwei antiparallele Magnetspulen 26d und 26e angeordnet. Das magnetische Feld der einen Spule treibt einen Eisenkern 26f, an dem der Schaft 2 mit einer Klemmutter 26g fixiert ist, in die eine Längsrichtung und die andere Spule den Schaft 2 in die entgegengesetzte Richtung. Der Schaft 2 kann somit in seiner Längsrichtung vibriert werden, wobei hierzu lediglich noch eine hier nicht gezeigte einfache elektrische Schaltung sowie ein Akkumulator als Spannungsquelle, der beispielsweise im Griff 26b untergebracht werden kann, erforderlich sind. Die Frequenz der Vibration liegt vorzugsweise im Bereich von 20-200 Hz. Die Amplitude des Kerns 26f liegt vorzugsweise bei etwa 2 mm. Infolge der Dämpfung des Schaftes ist die Amplitude A der Spitze 1 kleiner und liegt bei etwa 1 mm. Bei besonderen Anwendungen können die Amplitude und die Frequenz jedoch durchaus auch ausserhalb der genannten Bereiche liegen. Zur genaueren Steuerung der Vibration des Schaftes 2 wird vorzugsweise die Masse der Spulen 26d, 26e und des Gehäuses wesentlich höher gewählt als die Masse des Kerns 26f.

## Patentansprüche

1. Katheteranordnung zur Rekanalisation arterieller oder venöser und insbesondere koronarer Okklusionen, mit einem Führungsdraht, der einen Schaft (2) mit einem proximalen und einem flexiblen distalen Ende aufweist, wobei dieser Schaft (2) an seinem flexiblen Ende eine sich in Längsrichtung des Schaftes (2) erstreckende Spiralfeder (2d) sowie eine Spitze (1) aufweist, wobei der grösste Durchmesser (A) der Spitze (1) grösser ist als der Durchmesser (B) der Spiralfeder (2d) des Schaftes (2) unmittelbar hinter der Spitze (1), und mit einem Ballondilatationskatheter (3), der zur Einführung eines distal angeordneten Dilatationsballon auf dem Führungsdraht vorzuschieben ist, dadurch gekennzeichnet, dass die Spitze (1) des Führungsdrahtes olivenförmig ausgebildet und durch axiale Verschiebung des Dilatationsballons die Versteifung des distalen Endes des Führungsdrahtes veränderbar ist.

2. Katheteranordnung nach Anspruch 1, dadurch gekennzeichnet, dass der Schaft (2) einen am distalen Ende sich konisch verjüngenden Draht (2c) aufweist, der an seinem vorderen Ende (2d) fest mit der Spitze (1) verbunden ist und sich über die ganze Läge des Schafts (2) erstreckt, und dass der konische Bereich des Drahtes (2c) von der Spiralfeder (2d) umgeben ist, die am einen Ende an der Spitze (1) und am anderen Ende am Draht (2c) befestigt ist.

3. Katheteranordnung nach Anspruch 2, dadurch gekennzeichnet, dass der Draht (2c) in einem Bereich (2b) hinter der Spitze (1) flachgeschlagen ist.

4. Katheteranordnung nach einem der Ansprüche 2 oder 3, dadurch gekennzeichnet, dass die Spitze (1) aus Lot und insbesondere Silberlot hergestellt ist und das vordere Ende des Drahtes (2c) sowie das vordere Ende der Spiralfeder von der Spitze (1) umschmolzen sind.

5. Katheteranordnung nach einem der Ansprüche 1 bis 4, dadurch gekennzeichnet, dass die Spitze (1) einen grössten Durchmesser (A) von 0,7 - 3 mm, vorzugsweise etwa 1 mm aufweist.

6. Katheteranordnung nach einem der Ansprüche 1 bis 5, dadurch gekennzeichnet, dass der Durchmesser (B) des Schaftes (2) über seine ganze Länge etwa konstant ist und vorzugsweise etwa 0,5 mm beträgt.

7. Katheteranordnung nach einem der Ansprüche 1 bis 6, dadurch gekennzeichnet, dass das spitzere Ende der olivenförmigen Spitze (1) distal angeordnet ist.

8. Katheteranordnung nach einem der Ansprüche 1 bis 7, dadurch gekennzeichnet, dass die Spitze (1) eine zum Schaft (2) rotationssymmetrische Flankenfläche (1b) aufweist, die in einem Winkel (α) zur Schaftachse (2) vergleichsweise flach verläuft.

9. Katheteranordnung nach einem der Ansprüche 1 bis 8, dadurch gekennzeichnet, dass der Draht (2c) an seinem distalen Ende auf einer Länge von weniger als etwa 4 cm, vorzugsweise etwa 2 cm flach ausgebildet ist.

10. Katheteranordnung nach Anspruch 9, dadurch gekennzeichnet, dass der Draht (2c) im flachen Bereich im Querschnitt eine Breite D von etwa 0,1 mm und eine Dicke C von etwa 0,03 mm aufweist.

11. Katheteranordnung nach einem der Ansprüche 1 bis 10, dadurch gekennzeichnet, dass die Spitze (1) eine Länge von etwa 1,5 mm aufweist.

12. Katheteranordnung nach Anspruch 11, dadurch gekennzeichnet, dass der Ballon des Ballonkatheters (3) im dilatierten Zustand einen Durchmesser von 2 mm bis 4,25 mm aufweist.

13. Katheteranordnung nach Anspruch 11 oder 12, dadurch gekennzeichnet, dass der Führungsdraht rückwärts in eine distale Oeffnung (24a) des Ballonkatheters (3) eingeführt ist.

14. Katheteranordnung nach Anspruch 11, dadurch gekennzeichnet, dass der Schaft (2) mit einem Antrieb (26) verbunden ist, mit dem der Schaft (2) und die Spitze (1) zur Behandlung einer Okklusion in Längsrichtung des Schaftes vor und zurück bewegbar ist.

15. Katheteranordnung nach Anspruch 14, dadurch gekennzeichnet, dass der Schaft (2) und die Spitze (1) mit einer Frequenz von 20 bis 250 Hz bewegbar sind.

16. Katheteranordnung nach Anspruch 14 oder 15, dadurch gekennzeichnet, dass die Auslenkung der Spitze etwa 1 mm beträgt.

17. Verfahren zur Herstellung eines Führungsdrahtes nach Anspruch 1, dadurch gekennzeichnet, dass die Spitze (1) aus einem Stück Lot (20) gebildet wird, das am distalen Ende des Schaftes (2) angebracht und geschmolzen wird und anschliessend in einen festen Zustand gebracht wird und schliesslich zur Herstellung der geeigneten Form bearbeitet und insbesondere geschliffen wird.

18. Verfahren nach Anspruch 17, wobei der Führungsdraht einen sich am distalen Ende konisch verjüngenden Draht (2c) und an diesem Ende eine Spiralfeder (2d) aufweist, dadurch gekennzeichnet, dass mehrere Windungen am distalen Ende der Spiralfeder (2d) vor dem Anbringen des Lotstücks (20) axial gestreckt werden.

19. Verfahren nach Anspruch 17 oder 18, dadurch gekennzeichnet, dass das Lotstück (20) durch eine an dieses und an den Schaft (2) angelegte elektrische Spannung zum Schmelzen gebracht wird.

## Claims

1. Catheter arrangement for the recanalisation of arterial or venous and particularly coronary occlusions, having a guide wire comprising a shaft (2) with a proximal and a flexible distal end, this shaft (2) having, at its flexible end, a helical spring (2d) extending longitudinally of the shaft (2) and a tip (1), the maximum diameter (A) of the tip (1) being greater than the diameter (B) of the helical spring (2d) of the shaft (2) immediately behind the tip (1), and having a balloon dilatation catheter (3) which is to be advanced on the guide wire in order to insert a distally mounted dilatation balloon, characterised in that the tip (1) of the guide wire is olive-shaped and the stiffness of the distal end of the guide wire can be varied by axial movement of the dilatation balloon.

2. Catheter arrangement according to claim 1, characterised in that the shank (2) has a wire (2c) which tapers conically at the distal end, said wire (2c) being firmly attached to the tip (1) at its front end (2d) and extending over the entire length of the shank (2), and in that the conical region of the wire (2c) is surrounded by the helical spring (2d) which is attached at one end to the tip (1) and at the other end to the wire (2c).

3. Catheter according to claim 2, characterised in that the wire (2c) is flattened in an area (2b) behind the tip (1).

4. Catheter arrangement according to one of claims 2 and 3, characterised in that the tip (1) is made from solder and particularly silver solder and the front end of the wire (2c) and the front end of the helical spring are surrounded by the fused tip (1).

5. Catheter arrangement according to one of claims 1 to 4, characterised in that the tip (1) has a maximum diameter (A) of 0.7 - 3 mm, preferably about 1 mm.

6. Catheter arrangement according to one of claims 1 to 5, characterised in that the diameter (B) of the shank (2) is substantially constant over its entire length and is preferably about 0.5 mm.

7. Catheter arrangement according to one of claims 1 to 6, characterised in that the more pointed end of the olive-shaped tip (1) is located distally.

8. Catheter arrangement according to one of claims 1 to 7, characterised in that the tip (1) has a flank surface (1b) which is rotationally symmetrical relative to the shank (2) and which extends comparatively flatly at an angle (α) to the shaft axis (2).

9. Catheter arrangement according to one of claims 1 to 8, characterised in that the wire (2c) is of flat configuration at its distal end over a length of less than about 4 cm, preferably about 2 cm.

10. Catheter arrangement according to claim 9, characterised in that in the flat area the wire (2c) has a width D in cross section of about 0.1 mm and a thickness C of about 0.03 mm.

11. Catheter arrangement according to one of claims 1 to 10, characterised in that the tip (1) has a length of about 1.5 mm.

12. Catheter arrangement according to claim 11, characterised in that the balloon of the balloon catheter (3) has a diameter of 2 mm to 4.25 mm in the dilated state.

13. Catheter arrangement according to claim 11 or 12, characterised in that the guide wire is inserted backwards in a distal opening (24a) of the balloon catheter (3).

14. Catheter arrangement according to claim 11, characterised in that the shaft (2) is connected to a drive (26) by means of which the shaft (2) and the tip (1) can be moved back and forth in the longitudinal direction of the shaft in order to treat an occlusion.

15. Catheter arrangement according to claim 14, characterised in that the shaft (2) and the tip (1) are movable at a frequency of 20 to 250 Hz.

16. Catheter arrangement according to claim 14 or 15, characterised in that the deflection of the tip amounts to about 1 mm.

17. Process for producing a guide wire according to claim 1, characterised in that the tip (1) is formed from a piece of solder (20) which is applied to the distal end of the shaft (2) and fused thereon and is then brought into a solid state and finally machined and, in particular, polished in order to produce the appropriate shape.

18. Process according to claim 17, wherein the guide wire comprises a wire (2c) tapering conically at the distal end and at this end it comprises a helical spring (2d), characterised in that a plurality of windings at the distal end of the helical spring (2d) are stretched axially before the piece of solder (20) is applied.

19. Process according to claim 17 or 18, characterised in that the piece of solder (20) is made to fuse by an electric voltage applied to it and to the shaft (2).

## Revendications

1. Arrangement de cathéter destiné à la recanalisation d'occlusions artérielles ou veineuses et, en particulier, coronaires, avec un fil de guidage qui présente une tige (2) avec une extrémité proximale et une extrémité distale souple, cette tige (2) présentant, à son extrémité souple, un ressort hélicoïdal (2d) s'étendant dans le sens longitudinal de la tige (2) ainsi qu'une pointe (1), le diamètre le plus grand (A) de la pointe (1) étant plus grand que le diamètre (B) du ressort hélicoïdal (2d) de la tige (2) immédiatement derrière la pointe (1), et avec un cathéter de dilatation à ballon (3) qui doit être avancé sur le fil de guidage pour l'introduction d'un ballon de dilatation disposé distalement, caractérisé en ce que la pointe (1) du fil de guidage se présente en forme d'olive et que le raidissement de l'extrémité distale du fil de guidage peut être modifié par déplacement axial du ballon de dilatation.

2. Arrangement de cathéter suivant la revendication 1, caractérisé en ce que la tige (2) présente un fil (2c), s'effilant coniquement à l'extrémité distale, qui est, à son extrémité avant (2d), rendu solidaire avec la pointe (1) et s'étend sur toute la longueur de la tige (2) et que la partie conique du fil (2c) est entourée du ressort hélicoïdal (2d) qui est fixé, à l'une des extrémités, à la pointe (1) et, à l'autre extrémité, au fil (2c).

3. Arrangement de cathéter suivant la revendication 2, caractérisé en ce que le fil (2c) est aplati dans une zone (2b) derrière la pointe (1).

4. Arrangement de cathéter suivant l'une des revendications 2 ou 3, caractérisé en ce que la pointe (1) est réalisée en brasure et, en particulier, en brasure d'argent et que l'extrémité avant du fil (2c) ainsi que l'extrémité avant du ressort hélicoïdal sont entourées par fusion par la pointe (1).

5. Arrangement de cathéter suivant l'une des revendications 1 à 4, caractérisé en ce que la pointe (1) présente un diamètre le plus grand (A) de 0,7 à 3 mm, de préférence d'environ 1 mm.

6. Arrangement de cathéter suivant l'une des revendications 1 à 5, caractérisé en ce que le diamètre (B) de la tige (2) est environ constant sur toute sa longueur et est, de préférence, d'environ 0,5 mm.

7. Arrangement de cathéter suivant l'une des revendications 1 à 6, caractérisé en ce que l'extrémité la plus pointue de la pointe en forme d'olive (1) est disposée distale.

8. Arrangement de cathéter suivant l'une des revendications 1 à 7, caractérisé en ce que la pointe (1) présente une surface de flanc (1b), symétrique en rotation par rapport à la tige (2), qui s'étend comparativement à plat, selon un angle (α) par rapport à l'axe de tige (2).

9. Arrangement de cathéter suivant l'une des revendications 1 à 8, caractérisé en ce que le fil (2c) se présente plat, à son extrémité distale, sur une longueur de moins d'environ 4 cm, de préférence d'environ 2 cm.

10. Arrangement de cathéter suivant la revendication 9, caractérisé en ce que le fil (2c) présente, dans la partie plate, en section, une largeur D d'environ 0,1 mm et une épaisseur C d'environ 0,03 mm.

11. Arrangement de cathéter suivant l'une des revendications 1 à 10, caractérisé en ce que la pointe (1) présente une longueur d'environ 1,5 mm.

12. Arrangement de cathéter suivant la revendication 11, caractérisé en ce que le ballon du cathéter à ballon (3) présente, à l'état dilaté, un diamètre de 2 mm à 4,25 mm.

13. Arrangement de cathéter suivant la revendication 11 ou 12, caractérisé en ce que le fil de guidage est introduit à reculons dans une ouverture distale (24a) du cathéter à ballon (3).

14. Arrangement de cathéter suivant la revendication 11, caractérisé en ce que la tige (2) est reliée à un entraînement (26) à l'aide duquel la tige (2) et la pointe (1) peuvent être déplacées en avant et en arrière, dans le sens longitudinal de la tige, pour le traitement d'une occlusion.

15. Arrangement de cathéter suivant la revendication 14, caractérisé en ce que la tige (2) et la pointe (1) peuvent se mouvoir à une fréquence de 20 à 250 Hz.

16. Arrangement de cathéter suivant la revendication 14 ou 15, caractérisé en ce que la déviation de la pointe est d'environ 1 mm.

17. Procédé de fabrication d'un fil de guidage suivant la revendication 1, caractérisé en ce que la pointe (1) est réalisée à partir d'une pièce de brasure (20) qui est placée à l'extrémité distale de la tige (2) et fondue et qui est ensuite amenée à l'état solide et finalement usinée et, en particulier, polie, pour réaliser la forme appropriée.

18. Procédé suivant la revendication 17, le fil de guidage présentant un fil (2c) s'effilant coniquement à l'extrémité distale et, à cette extrémité, un ressort hélicoïdal (2d), caractérisé en ce que plusieurs spires à l'extrémité distale du ressort hélicoïdal (2d) sont étirées axialement avant le placement de la pièce de brasure (20).

19. Procédé suivant la revendication 17 ou 18, caractérisé en ce que la pièce de brasure (20) est amenée à fondre par une tension électrique appliquée sur celle-ci et sur la tige (2).
